(19) Europäisches Patentamt
European Patent Office
Office européen des brevets

(11) **EP 1 943 597 B1**

(12) **EUROPEAN PATENT SPECIFICATION**

(45) Date of publication and mention
of the grant of the patent:
**20.01.2010 Bulletin 2010/03**

(51) Int Cl.:
***G06F 15/173*** *(2006.01)*

(21) Application number: **06813583.9**

(22) Date of filing: **18.08.2006**

(86) International application number:
**PCT/US2006/032498**

(87) International publication number:
**WO 2007/022469 (22.02.2007 Gazette 2007/08)**

(54) **PARALLEL COMPUTER ARCHITECTURE FOR COMPUTATION OF PARTICLE INTERACTIONS**

PARALLELE COMPUTERARCHITEKTUR ZUR BERECHNUNG DER PARTIKELINTERAKTION

STRUCTURES DE CALCUL D'INTERACTIONS ENTRE PARTICULES

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.08.2005 US 709184 P**
**04.01.2006 US 756448 P**
**19.04.2006 PCT/US2006/014782**

(43) Date of publication of application:
**16.07.2008 Bulletin 2008/29**

(73) Proprietor: **D.E. Shaw Research, LLC**
**New York, New York 10036 (US)**

(72) Inventors:
• **DENEROFF, Martin, M.**
**Oakhurst, NJ 07755 (US)**
• **DROR, Ron**
**New York, New York 10023 (US)**
• **SHAW, David E.**
**New York, NY 10036 (US)**
• **LARSON, Richard H.**
**New York, NY 10028 (US)**
• **SALMON, John K.**
**Chappaqua,**
**NY 10514 (US)**

(74) Representative: **Cabinet Plasseraud**
**52, rue de la Victoire**
**75440 Paris Cedex 09 (FR)**

(56) References cited:
**US-B1- 6 600 788**

• **CULLER D E; PAL SINGH J: "Parallel computer architecture - a hardware/software approach" 1999, MORGAN KAUFMANN , SAN FRANCISCO, CA, US , XP002417055 ISBN: 1-55860-343-3 * section 1.2.7 & fig. 1.28 * * section 3.5.2 & fig. 3.16 * * section 7.7.1 & fig. 7.27 * * section 7.7.2 & fig. 7.29 * page 514, paragraph 3 * section 10.9.3 ***
• **CULLER D E ET AL: "Interconnection network design" PARALLEL COMPUTER ARCHITECTURE - A HARDWARE/SOFTWARE APPROACH, MORGAN KAUFMANN, SAN FRANCISCO, CA, US, 1999, pages 749-778, XP002333436**
• **CECCHET E: "Memory mapped networks: a new deal for distributed shared memories ? the SciFS experience" CLUSTER COMPUTING, 2002. PROCEEDINGS. 2002 IEEE INTERNATIONAL CONFERENCE ON 23-26 SEPT. 2002, PISCATAWAY, NJ, USA,IEEE, 23 September 2002 (2002-09-23), pages 231-238, XP010621880 ISBN: 0-7695-1745-5**
• **REINHARDT S K ET AL: "HARDWARE SUPPORT FOR FLEXIBLE DISTRIBUTED SHARED MEMORY" IEEE TRANSACTIONS ON COMPUTERS, IEEE SERVICE CENTER, LOS ALAMITOS, CA, US, vol. 47, no. 10, 1 October 1998 (1998-10-01), pages 1056-1072, XP000781991 ISSN: 0018-9340**
• **MUKHERJEE S S ET AL: "EFFICIENT SUPPORT FOR IRREGULAR APPLICATIONS ON DISTRIBUTED-MEMORY MACHINES" ACM SIGPLAN NOTICES, ACM, ASSOCIATION FOR COMPUTING MACHINERY, NEW YORK, NY, US, vol. 30, no. 8, 1 August 1995 (1995-08-01), pages 68-79, XP000528322 ISSN: 0362-1340**

- SHAW D E: "A fast, scalable method for the parallel evaluation of distance-limited pairwise particle interactions" INET, [Online] 12 July 2005 (2005-07-12), XP002417446 ISSN: 0192-8651 Retrieved from the Internet: URL:http: //www3.interscience.wiley.com/cgi -bin/fulltext/ 110562316/PDFSTART> [retrieved on 2007-01-30] cited in the application

- ORAL S ET AL: "A user-level multicast performance comparison of scalable coherent interface and myrinet interconnects" LOCAL COMPUTER NETWORKS, 2003. LCN '03. PROCEEDINGS. 28TH ANNUAL IEEE INTERNATIONAL CONFERENCE ON 20-24 OCT. 2003, PISCATAWAY, NJ, USA,IEEE, 20 October 2003 (2003-10-20), pages 518-527, XP010666014 ISBN: 0-7695-2037-5

## Description

Cross-Reference to Related Applications

This application claims the benefit of International Application Pub. No. WO/2006/113825. This application is also related to U.S. Application Pub. No. US2006/0129363A1, U.S. Application Pub. No. US2006/0142980A1, and International Application Pub No. WO/2006/004877A2.

Background

This document relates to computation of particle interactions, including algorithms and hardware and software techniques that are applicable to such computation.

Simulation of multiple-body interactions (often called "N-body" problems) is useful in a number of problem areas including celestial dynamics and computational chemistry. Biomolecular or electrostatic particle interaction simulations complement experiments by providing a uniquely detailed picture of the interaction between particles in a system. An important issue in - simulations is the simulation speed.

Determining the interaction between all pairs of bodies in the system by enumerating the pairs can be computationally intensive, and thus, alternate interaction schemes are often used. For example, all particles within a predefined radius of a particular particle are interacted with that particle while particles farther away are ignored. Special-purpose hardware has also been applied in order to reduce the overall computation time required for simulation.

US Pat. 6,600,788, "Narrow-band filter including sigma-delta modulator implemented in a programmable logic device," provides an example of a hardware implemented finite-impulse-response (FIR) filter, in which the filter coefficients are configurable to implement a sum of products algorithm.

Summary

In one aspect, in general, a computation system includes an array of processing modules arranged into one or more processing groups, each processing group including multiple serially interconnected processing modules. Each of the processing modules includes a storage for data elements and includes circuitry for performing pairwise computations between data elements, each of the pairwise computations making use of a data element from the storage of the processing module and a data element passing through the serially interconnected processing modules. Each of the processing modules also includes circuitry for selecting pairs of data elements according to separations between spatial locations associated with the data elements.

Aspects can include one or more of the following.

The array of processing modules includes two or more processing groups.

The system further includes distribution modules for distributing data elements among the processing groups.

The system further includes combination modules for combining results of the pairwise computations produced by each of the processing groups.

In another aspect, in general, a processing module for computing particle interactions in a multiple-body simulation is configured to perform pairwise computations between data elements. Each data element is associated with a corresponding spatial location. Each of the pairwise computations uses one data element from a first set of data elements and one data element from a second set of data elements. The processing module includes a set of matching units, each unit being associated with a storage for a different part of a first set of data elements. Each matching unit is configured to select pairs of data elements according to separations between the locations associated with the data elements. For each pair, one data element is from the first set and one data element is from a second set of data elements. The processing module also includes an input for receiving a series of data elements of the second set of data elements, the input being coupled to each of the matching units for passing the received data elements to the matching units. The module also includes a computation unit coupled to the matching units for receiving pairs of data elements selected by the matching units according to the separations, and an output for transmitting results produced by the computation unit.

Aspects can include one or more of the following features.

The module includes an output for transmitting the received series of data elements to another module.

The module includes an input for receiving results produced by other modules.

The module includes a merging unit for combining the results produced by the computation unit and the results received on the input for receiving results.

The merging unit includes circuitry for merging results according to a data element of the first set that is associated with each of the results.

In another aspect, in general, a method computing interactions in a multiple-body simulation includes receiving

## EP 1 943 597 B1

a first set of data elements, distributing the first set of data elements to the storages of an array of processing modules. The processing modules are arranged into one or more processing groups. Each processing group includes multiple serially interconnected processing modules. Distributing the first set of data elements includes storing a different part of the first set of data elements at each of the processing modules. The method includes receiving a second set of data elements, each of the data elements being associated with a corresponding spatial location. The data elements are passed through the serially interconnected processing groups. At each of at least some of the processing modules, some but not all of the received data elements are selected for the performing of the pairwise computations according to separations between spatial locations associated with the received data elements and spatial locations associated with the data elements distributed to the processing module. A pairwise computation is performed involving a data element from the first set that is stored in the storage of the processing module and a data element passing through the processing group, and a result of the pairwise computation is passed through the serially interconnected processing group. The method further includes combining the results of the pairwise computations passed from each of the processing groups and transmitting the combined results.

Aspects can include one or more of the following.

The data elements of the first set and of the second set are associated with bodies in a multiple body simulation system.

The pairwise computation is associated with a force between a pair of the bodies.

The method further includes, at each of the at least some of the processing modules, selecting some but not all of the data elements data elements passing through the processing group for the performing of the pairwise computations.

Selecting the data elements includes selecting the pairs of data elements for the performing of the pairwise computations.

Distributing the first set of data elements to the storages of the array includes, at each of the modules, distributing data elements to a plurality of matching units, each matching unit receiving a different subset of the data elements.

The method further includes at each of the at least some of the processing modules at each of the matching units selecting some but not all of the data elements data elements passing through the processing group for the performing of the pairwise computations.

The method further includes at each of the at least some of the processing modules combining the outputs of the matching units, and passing the combined outputs to a computation unit for executing the pairwise computation.

In another aspect, in general, a software product is adapted to perform all the steps of any of the above methods. The software product can include instructions for performing the steps embodied on a computer-readable medium.

Other aspects and features are apparent from the following description and from the claims.

Description of Drawings

FIG. 1 is a table of asymptotic scaling for various decomposition methods.

FIG. 2 is perspective view of regions for the Half-Shell (HS) method.

FIG. 3 is perspective view of regions for the Neutral Territory (NT) method.

FIG. 4 is perspective view of regions for the Snir's Hybrid (SH) method.

FIG. 5 is a block diagram of a parallel implementation.

FIG. 6 is a flowchart.

FIG. 7 is a block diagram including elements of a processing node.

FIG. 8 is a diagram illustrating data flow in a processing iteration.

FIG. 9 is a functional block diagram of a particle-particle interaction module (PPIM).

FIG. 10 is a block diagram of a portion of a midrange subsystem.

FIG. 11 is a block diagram of an implementation of a PPIM.

FIG. 12 is a block diagram of a flexible subsystem.

Description

Overview

Simulation of multiple-body interactions (often called "N-body" problems) is useful in a number of problem areas including celestial dynamics and computational chemistry. For example, biomolecular or electrostatic particle interaction simulations complement experiments by providing a uniquely detailed picture of the interaction between particles in a system. A significant issue in simulations is the simulation speed.

1.1 Molecular dynamics

One approach to molecular simulation is primarily physics-based, predicting structure based on a mathematical model of the physics of a molecular system. Proteins may be modeled at the atomic level, with individual atoms or groups of atoms represented as point bodies in an N-body system. One of these methods is molecular dynamics (MD), in which the force on each particle is calculated and Newton's laws are numerically integrated to predict the physical trajectory of each atom over time. An alternative is a class of Monte Carlo methods, which stochastically sample the potential energy surface of a system. Physics-based methods can either be used to refine homology models or be applied on their own, to determine protein structure from scratch. MD can also be used to study the process of protein folding.
One factor in applying physics-based methods to protein structure prediction and other structure-based problems in computational biochemistry is that often these problems are not concerned with any single molecule or molecular event, but rather with the statistical properties of a very large collection of molecules. For instance, when predicting protein structure, we may not necessarily be interested in the structure that one particular protein molecule may happen to fold into, but rather the most probable structure for molecules of that protein, the structure that will be the norm among a large collection of molecules of that protein. When studying binding, the question may not be whether a particular protein and ligand will bind in a particular instance, but the concentration of bound ligands in the steady state, when a large number of proteins and ligands are put together.
A useful quantity to compute for these problems is the free energy of a molecular system. Free energy is not a property of a single state of a system, but rather of an ensemble of states. In very general terms, the lower the free energy of an ensemble of states, the higher the probability that a molecular system will be found in one of those states at any given time. The free energy of an ensemble is computed by summing probabilities over all states in the ensemble. The probability of a state is an exponential function of its potential energy that depends on the temperature of the system (specifically, the calculation uses a Boltzmann distribution). In practical terms, this means that for protein structure prediction, it is insufficient to perform a single run of an MD simulation, stop at some point, and take the end conformation as the native state. At a minimum, many conformations near the end of such a simulation should be sampled. More likely, multiple separate simulations may need to be run and the results compared.
Once the structure of a target protein has been obtained-through either computational or experimental methods-computational methods can be used to predict the protein's interactions with ligands, a task loosely referred to as "the docking problem."
Screening ligands for activity could in theory be done with physics-based methods. Owing to the enormous computational burden of that approach, other methods have been developed. Generally, different configurations of a protein and ligand are generated and a score is calculated for each according to a heuristic scoring function. To reduce the number of configurations that must be tested, the protein is held rigid or allowed a small degree of flexibility; the ligand is tested in various poses and orientations at various points near the protein. To further reduce the configuration space, it is necessary (or at least very helpful) to know the active site on the protein.
This heuristic screening may identify ligands that are likely to bind to a target protein, but it may not quantify that binding or predict other properties of the interaction, such as the concentration of the ligand necessary to produce a certain effect. For this purpose, the binding free energy of the interaction, the free energy difference between the unbound and bound states of the system, can be used. Too small a binding energy can make the required dosage of a drug too high to be practical; too high an energy can cause toxicity or other side effects. Accurate calculation of binding free energy is a significantly more computationally intensive task than simply determining whether two molecules are likely to bind, and relies heavily on accurate structural models of these interactions.

1.2 Molecular force fields

In physics-based methods for structure-based problems, the "inner loop" of these computational methods can be chosen to solve the following problem: given a molecular system, calculate either (a) the potential energy of the system as a whole or (b) the force on each particle due to its interactions with the rest of the system. (Note that the force in (b) is simply the three-dimensional vector gradient of (a), making the two variations of the problem similar computationally.) These forces and energies are generally computed according to a particular type of model called a molecular force field.

Force fields can model molecular systems at the atomic level, with atoms or groups of atoms typically represented as point bodies. Each point body has a set of associated parameters such as mass and charge (more specifically a partial charge, so that the complicated electron distribution caused by atomic bonding can be modeled with point charges). These parameters are determined at initialization according to the atom's type and, in standard force fields, remain constant throughout the simulation. Atom type is not as simple as an atomic number on the periodic table: an atom's parameters can depend upon what particles are near it and, in particular, what other atoms are bonded to it. For example, a hydrogen atom bonded to a nitrogen atom (an amine) may have a partial charge that is different than one bonded to an oxygen atom (an alcohol). There can easily be several types each of carbon, hydrogen, oxygen, and nitrogen. The set of atom types and the parameters used for them are one of the characteristics that define a particular force field.

The interactions among atoms can be broken into several components. The bonded terms model the interaction between atoms that are covalently bonded. One such term effectively models a bond between two atoms as a harmonic oscillator, reflecting the tendency for two atoms to settle at a certain distance from each other, known as the bond length. Another term reflects the tendency for two bonds to "bend" towards a certain angle. Yet another term takes into account the effect of torsion, the "twisting" of a bond owing to the relative angles it makes with two bonds on either side of it. Several other types of terms are possible, many of them cross-terms, which take into account the interaction between the basic types of terms. Each term contains one or more parameters, and each parameter must have a value for each combination (pair, triplet, quartet, etc.) of atom types, leading to a profusion of parameters from all of the terms and cross-terms.

The non-bonded terms in a force field model all-to-all interactions among atoms. One of the non-bonded terms calculates the electrostatic interaction of charges attracting and repelling each other according to Coulomb's law. Another type of non-bonded term models the van der Waals interaction, a shorter-range interaction that comprises an attractive component and a repulsive component. The repulsive component dominates at very short distances of a few angstroms (abbreviated Å, equal to $10^{-10}$ meters). In a rough sense, the repulsive force can be thought of as keeping atoms from overlapping or crashing into one another. There are various ways to model the van der Waals potential. The attractive component is usually modeled as dropping off as the inverse sixth power of the distance between two particles, or $1/r^6$; the repulsive component can be modeled with a similar power function (usually $1/r^{12}$, for computational convenience on general-purpose processors) or with an exponential. Such forces are in contrast the electrostatic potential which drops off more slowly, as $1/r$.

1.3 <u>Pairwise interactions</u>

Simulations in many fields require computation of explicit pairwise interactions of particles. In addition to the molecular dynamics and Monte Carlo simulations in biochemistry discussed above, similar simulations may be applied to materials science, N-body simulations in astrophysics and plasma physics, and particle-based hydrodynamic and equation of state simulations in fluid dynamics. To reduce the computational workload of such simulations, instead of computing interactions between all pairs of particles, one approach is to compute interactions only between pairs separated by less than some *interaction radius, R.* These interactions are referred to as "near interactions." The remaining interactions ("distant interactions") are either neglected or handled by a less computationally expensive method. Even with the savings associated with limiting the interaction radius, the near interactions still frequently dominate the computational cost. Methods for decomposing this computation for parallel and/or serial processing can be useful in reducing the total time required and/or computation or communication required for these simulations.

Traditional methods for parallelization are described in a number of review papers, including Heffelfinger, "Parallel atomistic simulations," Computer Physics Communications, vol. 128. pp. 219-237 (2000), and Plimpton, "Fast Parallel Algorithms for Short Range Molecular Dynamics," Journal of Computational Physics, vol. 117. pp. 1-19 (March 1995). They include atom, force, and spatial decomposition methods. Spatial decomposition methods can offer an advantage over atom and force decomposition methods because they can exploit spatial locality to only consider pairs of nearby particles rather than all pairs in the system. Force decomposition methods, on the other hand, can offer an advantage over spatial and atom decomposition methods in that the communication bandwidth required by each processor decreases as the number of processors increases.

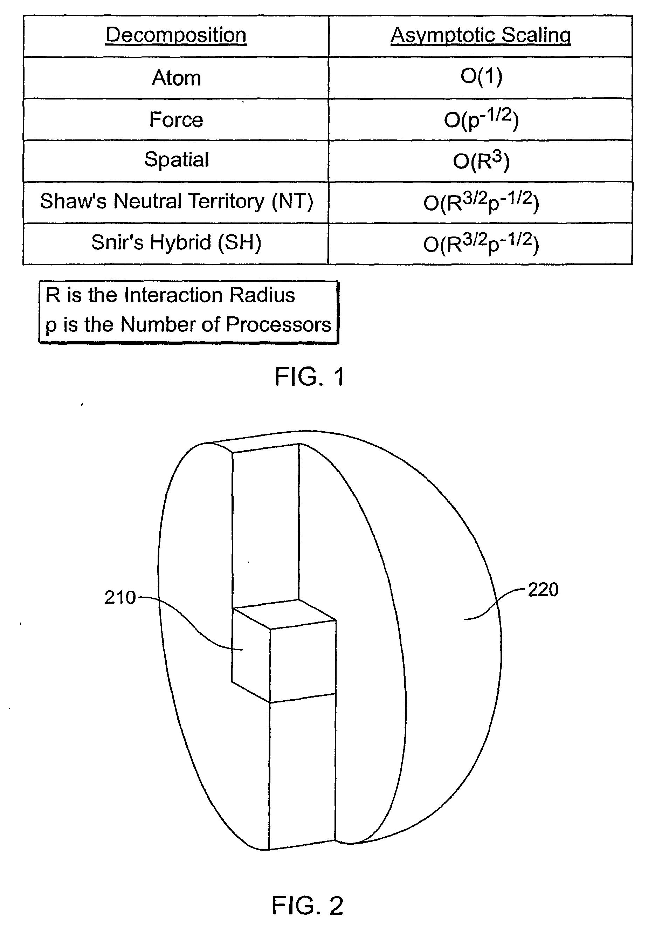

Two recently developed methods - Snir's hybrid (SH, "hybrid") method, published as "A Note on N-Body Computations with Cutoffs," by Marc Snir (Theory Comput. Systems 37, 295-318, 2004) and Shaw's Neutral Territory (NT) method, to be published as "A Fast, Scalable Method for the Parallel Evaluation of Distance-Limited Pairwise Particle Interactions" by David E. Shaw, combine spatial and force decompositions. They have a property that the communications required decreases as the interaction radius decreases. They also have a property that the communication required per node decreases as the number of processors increases. Referring to FIG. 1, a comparison of asymptotic scaling properties of the SH and NT methods with those of traditional methods shows that the SH and NT methods have the same scaling properties, which are significantly better than those of traditional methods. Specifically, both of these two methods have asymptotic complexity of $O(R^{3/2}p^{-1/2})$, which grow more slowly with the number of processors, p, than all but the force method, which does not exploit spatial locality and therefore does not improve as the interaction radius, R, is reduced.

Both the SH and NT methods can offer advantages over traditional methods for practical machine and simulation sizes. For instance, the NT method can be shown to always requires less communication that the SH method, but that the SH method can be optimized using features of the NT method such that the difference was small. An optimized version of the SH method that makes use of features of the NT method is referred to below as the SNT method.

The NT, SNT, and SH methods involve different *import regions.* That is, the set of particles imported by each processor differs from one method to another. The methods, however, share a number of features. In both the NT method and the SH method, each processor stores the particles falling in some rectangular box. In both methods, the processor that computes the interaction between a pair of particles typically is not the one on which either particle resides, but instead a third processor that imports both of the particles of the pair. In both methods, each processor imports particles from two *interaction zones* and considers all pairs of particles such that one particle is in one interaction zone and the other particle is in the other interaction zone.

The description below addresses a general class of methods that includes traditional spatial decompositions, the SH method, the NT method, and the SNT method as special cases. The communication requirements of a variety of specific instances of the general class are analyzed taking into account both latency and bandwidth of the interprocessor communication network.

A criterion for selecting parameters of a particular method can depend on the properties of both the physical system to be simulated (e.g., system dimensions, interaction radius) and the hardware available for the simulation (e.g., number of nodes, network topology, and memory structure including cache structure and sharing between nodes). For most practical parameter settings, the method that delivers the best performance according to such a criterion generally is not necessarily one of the specific methods described above, but rather is a different instance of the general class of methods.

## 1.4 Mesh based computations

As introduced above, distant interactions between particles, for example, between particles separated by more than the maximum interaction radius, can be computed using less computationally expensive methods. One such approach is based on the Ewald Method, as described in detail in copending applications U.S. Application Pub. No. US2006/0129363A1, U.S. Application Pub. No. US2006/0142980A1, and International Application Pub. No. WO/2006/004877A2.

The essence of the Ewald method is to split the electrostatic potential field into two main parts, one of which can be computed efficiently in real space (by calculating pairwise interactions) and the other of which can be handled efficiently in the Fourier domain using computations on a regular grid on the simulation region. (Note, however, that not all variations on Ewald use the Fourier domain.)

Implementation of the part involving the Fourier domain calculations can be divided into three phases. First, a discrete charge distribution

$$\rho(\mathbf{r}) = \sum_{i=1}^{N} \sum_{\mathbf{n}} q_i \delta(\mathbf{r} - \mathbf{r}_i - \mathbf{n}L)$$

that is defined on the (infinite and periodic) continuous three-dimensional space r, is mapped to a charge distribution $\rho^S(\mathbf{r}_m)$ defined on the finite three-dimensional mesh according to

$$\rho^S(\mathbf{r}_m) = S \otimes \rho = \int_{-\infty}^{\infty} S(\mathbf{r}_m - \mathbf{r})\rho(\mathbf{r})d^3\mathbf{r} = \sum_{i=1}^{N} \sum_{\mathbf{n}} q_i S(\mathbf{r}_m - \mathbf{r}_i - \mathbf{n}L) \approx \sum_{i=1}^{N} q_i S(\mathbf{r}_{im}^{min})$$

where $S$ is a charge spreading function, the operator $\otimes$ denotes spatial convolution, $m$ is an index for mesh points that lie within the primary box, and the vector $\mathbf{r}_{im}^{\min}$ is the vector from mesh point $m$ to the nearest image of particle $i$.

In a second phase, a potential function $\phi^*(\mathbf{r}_m)$ is computed at the grid locations using a convolution operation on the charge distribution $\rho^S(\mathbf{r}_m)$. This convolution can be implemented using Fast Fourier Transform (FFT) techniques.

In the third phase, energy or force quantities defined at the particle locations are found using a convolution approach that maps the mesh-based potential function to those locations. In particular, the force on the $i^{th}$ particle can be computed as

$$\mathbf{F}_i^{\sigma} = q_i \mathbf{F}^{\sigma}(\mathbf{r}_i)$$

where $\mathbf{F}^{\sigma} = T' \otimes \phi^*$ and $\mathbf{T}'(\mathbf{r}) = \dfrac{d}{d\mathbf{r}} T(\mathbf{r})$ . This third phase can be implemented as a sum

$$\mathbf{F}_i^{\sigma} = q_i h^3 \sum_{m(\mathbf{r}_i, T)} \phi^*(\mathbf{r}_m) \mathbf{T}'(\mathbf{r}_{im}^{\min}) \ .$$

Note that computationally, the first phase and the third phase involve sums involving pairs of locations, with one location of each pair being a particle ($i$) location and the other location of the pair being a location of a mesh point *(m)*.

1.5 <u>Document organization</u>

The remainder of this document is organized as follows. Section 2 generally relates to algorithms that are applicable to computing range-limited pairwise interactions between particles, or more generally, to performing range-limited computations associated with pairs of points in space, where the pair may correspond to a pair of particles in an N-body problem, or the pair may correspond to one grid point and one particle point, for example, as part of a charge spreading or force interpolation method.

Section 3 relates to hardware techniques, which may be applicable to the algorithms presented in Section 2, or to an overall n-body simulation implementation. Note that the hardware techniques described, for example in Section 3, have aspects that are applicable to a much broader range of applications than n-body simulation.

Possible applications of the algorithms and software and hardware techniques are discussed briefly in Section 4.

2 <u>NT, SH, and HS Methods</u>

The recently presented NT and SH methods, as well as an example of a conventional spatial decomposition method, the "Half-Shell" or HS method, can be described in the following framework. We assume that the system of particles (bodies) being simulated resides within a rectangular box called the *global cell.* In all three methods, we divide the global cell into a regular, rectangular, three-dimensional grid of smaller boxes. We refer to these smaller boxes as *homeboxes.* In one parallel implementation, we assign a one-to-one correspondence between homeboxes and processors, and we refer interchangeably to a processor and its homebox. The methods are described below in the content of such an implementation, with some alternative implementations being presented after this discussion.

Also, when we refer to a homebox or processor importing particles, this should be understood to include a processor receiving information characterizing those particles, such as their location, physical attributes, identities, etc. Each processor is responsible for updating the position of the particles that lie within its homebox at any point in time. As a convention, the *base coordinates* of a given home box (or of any particle within that homebox) are defined as the coordinates of the low-coordinate corner of that homebox. The *logical coordinates* of a homebox or particle are the integer mesh coordinates of the homebox. The dimensions of each homebox are $h_x \times h_y \times h_z$.

Each home box has an associated *import region* consisting of the region of space from which the processor must import particles. Each home box imports all particles not already present within that homebox that are to be interacted in the home box. The NT, SH, and HS methods each use different import regions.

The NT, SH, and HS methods each interact all particles in one volume of space with all particle in another volume of space. We refer to these volumes as "interaction zones" or simply "zones." In each of these methods, the intersection of the two zones is the homebox.

We use the volume of the import region associated with each homebox as a simple model for (i.e., a proxy for or an estimate of) the communication bandwidth required by pairwise particle interaction method. Such a proxy is most accurate for a fully connected network topology.

### 2.1 HS method

In conventional decompositions, generally, two particles are interacted in the homebox in which one of them resides. In the HS method specifically, the location where a pair of particles are interacted follows the following rule: the particles are interacted in the homebox with the smaller x base coordinate; if the two particles have the same x base coordinate; the particles are interacted in the homebox with the smaller y base coordinate; if they also have the same y base coordinate, they are interacted in the homebox with the smaller z base coordinate. If they also have the same base z base coordinates, they are in the same homebox and the interaction is computed there. Interactions between particles on the same homebox are referred to as "local interactions" while interactions between particles on different homeboxes are referred to as "remote interactions."

Referring to FIG. 2, as a result of the rule, each homebox has an import region that consists of half the points external to the homebox within a distance R of the boundary. Every particle in the import region 220 interacts with every particle in the homebox 210. Every particle in the homebox 210 also interacts with every other particle in the homebox 210. Equivalently, the two interaction zones are the homebox and the union of the homebox with the half shell.

The conventional HS method is guaranteed to consider all unique pairs separated by less than R. The method also could interact some pairs whose separation exceeds R due to the finite volume of the homebox. These "excess interactions" can be eliminated ("filtered") at the pair level as necessary. Local interactions will also need filtering to avoid double counting interactions between local particles. Such filtering can be accomplished by a test within an inner loop of a nested computational iteration of particles.

### 2.2 NT method

In the NT method, two particles interact in a homebox that has the $x$ and $y$ base coordinates associated with one particle and the $z$ base coordinate associated with the other particle. More specifically, the $x$ and $y$ base coordinates of the homebox in which the particles interact are those of the particle with the smaller $x$ base coordinate or, if both particles have the same $x$ base coordinate, those of the particle with the smaller $y$ base coordinate. The $z$ base coordinate of the homebox in which the particles interact is the z base coordinate of the other particle. If two particles have the same $x$ and $y$ base coordinates, they are interacted on the homebox of the particle with the lower $z$ coordinate.

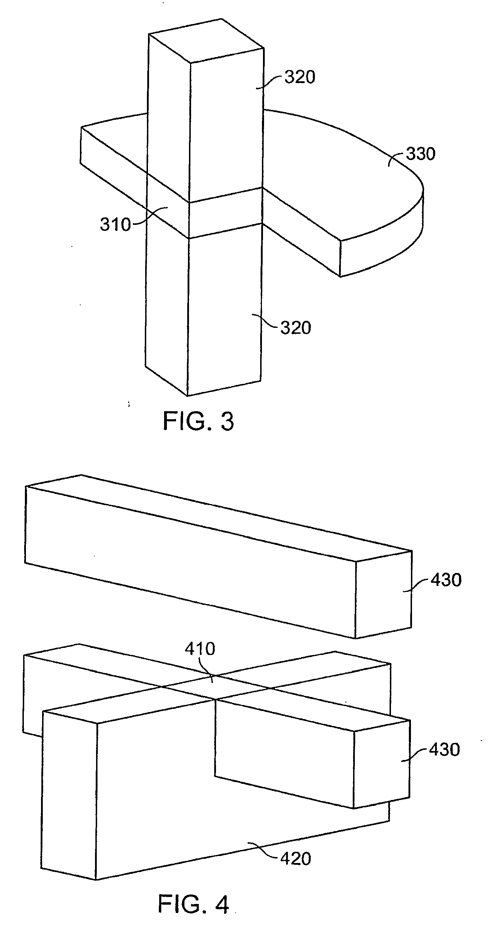

Referring to FIG. 3, the resulting import region of each homebox is the union an "outer tower" 320 and an "outer plate" 330. The outer tower comprises all points that lie directly above or below the homebox 310 within a distance $R$ of the boundary. The outer plate 330 comprises half the points external to the homebox 310 that lie in its z-plane within a distance R of the boundary. The interaction zones are the "tower," which consists of the union of the homebox 310 and the outer tower 320, and the "plate," which consists of the union of the homebox 310 and the outer plate 330.

By interacting the tower (310, 320) with the plate (310, 330), all near interactions are guaranteed to be computed at least once. However, to ensure that each near interaction is computed only once, a filtering criterion is used that is not required in the HS method. Namely, local (i.e., homebox) plate particles should only interact with local tower particles and remote (i.e., non-homebox) particles in the upper half of the outer tower (or lower half, but not both).

The import requirements of the NT method can be reduced by an appropriate choice of the aspect ratio of the homebox appropriately. An optimum occurs when $h_x = h_y > h_z$. That is, an optimum occurs when the homeboxes are "flattened" in the z dimension by an amount that depends on the interaction radius and the homebox volume, and generally corresponds to the tow interaction zones having equal or approximately equal volumes.

### 2.3 SH method

The SH method assumes a cubical homebox of side length $h$. We define $r = \lceil R / h \rceil$ and $\tilde{r} = \left\lceil \sqrt{r + 1} \right\rceil$.

Referring to FIG. 4, the import region of the homebox 410 with logical coordinates $(i, j, k)$ (including the homebox) consists of the union of two interaction zones. The first interaction zone is a contiguous region that we refer to as the "base" 420 and consists of the set of homeboxes with logical coordinates $(i + u, j, k - w_1)$ where $u \in [-r, r]$ and $w_1 \in [0, \tilde{r} - 1]$. The second interaction zone is a set of non-contiguous regions that we refer to as the "comb" 430. It consists of the set of boxes with logical coordinates $(i, j + v, k + w_2\tilde{r})$, where $v \in [-r, r]$ and $w_2 \in [0, Lr/\tilde{r}]$. Note that both the comb 430 and the base 420 include the homebox 410.

Interacting the comb 430 and base 420 ensures that all near interactions will be computed at least once. As for the HS and NT methods, filtering can be used to ensure that all near interactions are computed exactly once and that only near interactions are computed.

In a generalized class of methods, each processor imports particles from two interaction zones and interacts particles from one zone with particles from the other zone. The pairs of zones are chosen for the implementation of the method to ensure that all near interactions are computed. Both the case where each particle interacts with other particles in a surrounding cube, and the case where each particle interacts with other particles in a surrounding sphere are considered. Because a spherical region of radius $R$ has roughly half the volume of the circumscribing cube of side length $2R$, "rounding" allows reduction in communication bandwidth requirements.

3 Parallel implementation

One approach to computation of particle interactions, which can include one or more of the algorithmic techniques described above, makes use of special-purpose hardware that is particularly suitable for various computations necessary for computing particle interactions. The special purpose hardware is arranged in a parallel architecture that includes multiple processing nodes, with each processing node being responsible for the computations associated with one homebox. The architecture permits internode communication. One arrangement of nodes is in a three-dimensional array in which each node communicates directly with its six neighbors along three orthogonal axes, and communication of particle data can involve passing the data through multiple nodes to reach its destination.

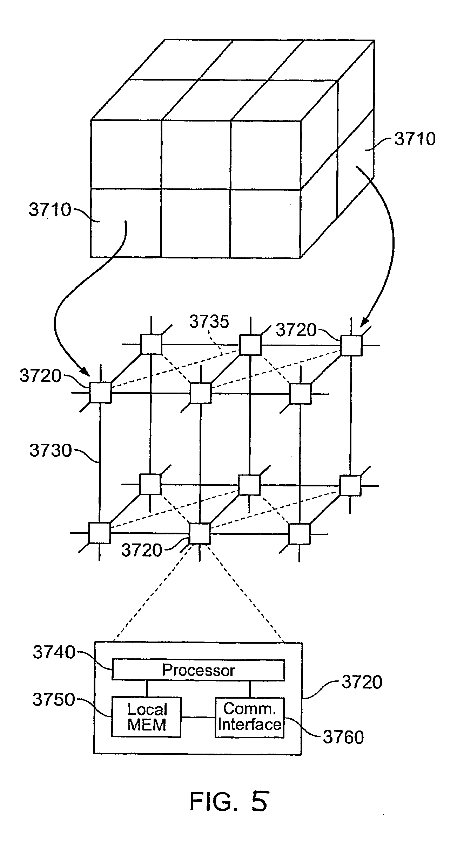

Referring to FIG. 5, a representative three-dimensional array of computation nodes 3720 is arranged such that each node performs the computation for a corresponding homebox 3710. The nodes are linked by communication paths 3730 along the orthogonal axes, and optionally by diagonal communication paths 3735. Each processing node 3720 includes a processor 3720, which performs numerical calculations associated with the particle interactions, and a communication interface 3750, which is responsible for sending and receiving particle data over communication links 3730, 3735 linking the node to its neighboring nodes.

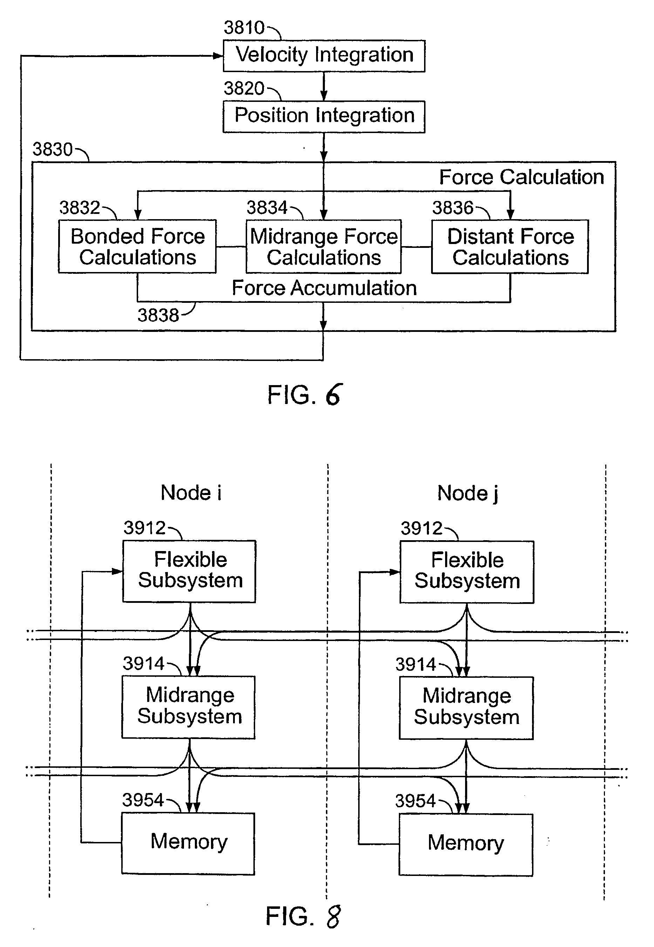

Before discussing the details of the hardware implementation, the computation performed by the hardware can be understood with reference to FIG. 6. The computation involves a loop that is performed at each time step of the simulation, with each time step representing a simulated time such as 1 or 2 femtoseconds (abbreviated fs, equal to $10^{-15}$ seconds). Generally, each iteration involves three basic step. Velocity integration 3810 uses the calculated forces on each particle to update the velocity of that particle. Position integration 3820 uses the updated velocities to calculate the updated positions of the particles. Force calculation 3830 uses the new positions of the particles to calculate forces on the particles, thereby allowing the iteration to repeat.

Force calculation involves calculations at different spatial scales. These calculations are performed independently. There calculations include bonded force calculation 3832, midrange force calculation 3834, and distant force calculation 3836. As part of each of these calculations, force accumulation 3838 determines the total force on each particle, with the force on any particular particle potentially including components that are determined by computations at each of the spatial scales. It is not necessary to perform the force calculations at each of the spatial scales at each time step. The distant force computations may be performed less frequently than the midrange computations, which are performed less frequently than the bonded force calculations, for example, using a MTS (Multiple Time Scale) and/or RESPA (REference System Propagator Algorithm) approach.

Another aspect of the parallel implementation involves dedicating different hardware elements to various types or spatial scales of computations. For example, hardware elements are dedicated to computations involving pairwise particle interactions that are part of the midrange force calculation step 3834 (or particle-grid computations that form part of the distant force calculations 3836), and to computations of near interactions such that are part of the bonded force calculations 3832. In addition the force accumulation step 3838 makes use of a memory system that incorporates numeric value accumulation functionality. In the multiple node architecture introduced above, each node includes such dedicated hardware elements for the various types of computations.

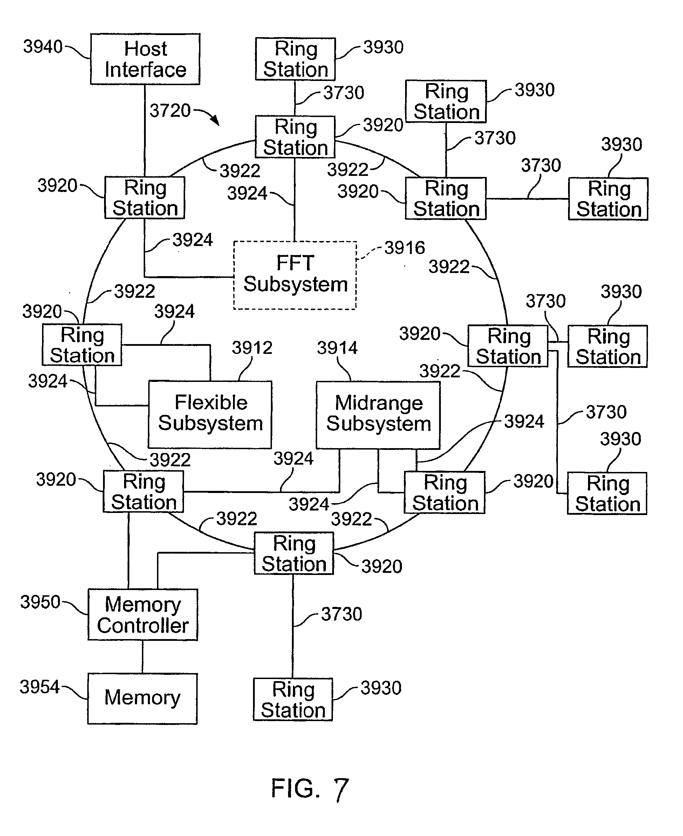

Referring to FIG. 7, each computation node 3720 includes a number of computation and communication elements. A flexible subsystem 3912 includes one or more programmable processors. A midrange system 3914 includes special-purpose circuitry that is adapted to perform pairwise computations, for example, between pairs of particles. An FFT (Fast Fourier Transform) subsystem 3924 is used in some embodiments to perform computations related to distant force computations. Yet other embodiments may include additional or fewer specialized or general purpose computations elements.

Communication within one processing node makes use of a ring communication architecture that includes a number of ring stations 3920 coupled by communication links 3922. In this version of the system, there are eight ring stations per node, and the links are high speed serial communication links. Each ring station has additional links. For example links 3924 couple the ring stations to the computation elements of the node. Other links 3730 couple ring stations for this node to ring stations 3930 of the six neighboring processing nodes 3720 of the torroidal mesh of processors. Other links couple the ring stations to a memory controller 3950, which provides access to a memory 3954, and to a host interface 3940, which is used for example for administrative functions such as loading programs to be executed on the computation elements.

The communication architecture, which is made up of the interconnected communication rings on each node, supports a global addressing approach by which each computation or communication element coupled to a ring station can address a data packet to any other element in the system, which may be on the same node, or may be on another node. Also, as is described further below, the communication architecture supports forms of limited multicast that allows data packets sent from a source to be distributed to multiple destinations without necessarily broadcasting the packet to all nodes in the system or sending multiple copies of the data packet from the source node.

In general, implementation of the iteration illustrated in the flowchart of FIG. 6 using the node architecture illustrated in FIG. 7 involves partitioning tasks among nodes, and partitioning the processing steps among the computation elements of each node. Furthermore, each of the subsystems takes advantage of internal parallelism. Overall, the implementation provides parallel processing and pipelining of computations at multiple levels of the architecture.

Referring to FIG. 8, computation elements for two nodes are illustrated, with indications of some of the data flows between the elements in a computation iteration. The communication links within a node and between nodes are not illustrated. The data flow that is illustrated relates to execution of the velocity and position integration steps (3810, 3820 in FIG. 6) as well as the midrange force calculation 3834. Other data flows, for example, related to distant forces and bonded forces are not illustrated in this figure.

Generally, at one point of the iteration time step, the flexible subsystem 3912 on each node retrieves particle force data for particles it is responsible for from its own memory 3954. Using the force data, it performs the integration steps required to determine the new particle positions. This particle position data does not have to be returned to the memories 3954. Rather, each flexible subsystem 3912 multicasts its position data, and this position data is passed to those midrange systems 3914 that will use the data to compute pairwise particle interactions, for example, using the NT method described above. In particular, the multicast approach ensures that the midrange subsystem of each node receives the particle data for the entire import region needed for the decomposition approach being used. Furthermore, data is "pushed" to the consumer of the data without necessarily requiring requests or acknowledgements for the data, thereby reducing communication load and delay associated with such communication.

In a pipelined manner, the midrange subsystems 3914 begins computing pairwise interactions as they receive the required particle data. For example, the midrange subsystem 3914 at a node may receive the particle position data for particles in that node's homebox relatively quickly over on-node communication paths, and then start receiving position data from neighboring nodes.

As the midrange system 3914 computes force contributions for particles, it distributes these contribution terms to the memories 3954 at the nodes responsible for those particles. The memories include accumulation functionality that allows the memory on sum the force contributions on each particle as the force contributions for that particle are received from various nodes. Using the accumulation function of the memories, different processors can compute components of a sum (e.g., of forces) and send them to a memory where the components are accumulated. Because the communication of the components does not require acknowledgement from the memories to the processors that produced the components, efficient accumulation is achieved. The memories implement the accumulation so that it is not order-dependent so that the identical result is obtained regardless of the order in which components to be summed together arrive at the memory.

Not illustrated in FIG. 8 are similar pipelined parallel procedures for the distance force computations, which involve grid-based computations that make use of Fast Fourier Transform (FFT) techniques that use multiple interchanges of data between nodes, and in some embodiments makes use of the specialized FFT subsystem 3916 to implement the FFT computations.

Referring again to FIG. 7, the communication system supports packet-based message interchange between elements on the same or on different nodes. Packets can represent operations including read, write, accumulate, and read-modify-write with the packet header specifying the operation and the packet body carrying operands (e.g., values to the written). A global address space is used for registers, memory locations, etc. in any node, with a 48-bit address space having 16 bits reserved for node number and the remaining 32 bits for 4G address locations (e.g., bytes) at each node. Different address ranges at a node map to different ring stations on the node based on routing information stored in the ring stations, and at each ring station, different address ranges are mapped to different subsystems coupled to the ring station. Packets are variable length having between one (i.e., a header only) and nine units of length (a header plus eight units of data), with each unit being, for example, 256 bits.

Each ring station is programmable with respect to routing, and is configured for a particular routing pattern before the simulation iterations are started. Each ring station includes a configuration table that is loaded before processing begins. In some examples, this table specifies a set of production rules that are applied to each incoming data packet. In general, the table in each ring stations specifies how packets that arrive at that ring station should be handled. Each packet that comes in is passed on one (or more) of the other ports (which can send the data off to another node, or on its way to a module on the same node) according to the table based on the source or destination address, and optionally based on a packet type. The configuration table can also specify an address translation function to be performed on the data before it is forwarded.

Every ring station in this embodiment has two ports for communication on the ring, and two client ports, for example, for connecting to ring stations at other nodes or for connecting to computation elements at the node.

Typically, messages that are sent from client to client are not acknowledged. However, for some communication an acknowledgement may be generated by the destination element and used for synchronization purposes. This embodiment does not perform error correction if there are communication errors for example by retransmission. Rather, the computation is restarted from a previously checkpointed timestep. Checkpoints are generated periodically, for example, with a period ranging from approximately one second to one minute of real time.

One use of the multicast mechanism is for distribution of various types of data to nodes according to the type of data. For example, depending on the import region for particle data, the processors that need copies of particle data from one processor is determined by the geometry of the import region. The ring stations of the communication system are configured such that the source processor for the particle data does not need to be aware to the communication interconnections across which the particle data must pass, or where copies of the data are to be made. Rather the source processor tags the data with a group id corresponding to the type of data, and the communication system distributes it to all processors that need to import than data according to the configuration of the communication system.

A multicast mechanism is provided for distributing position data, for example, in the implementation of the NT method discussed above. Multicast is implemented using a limited range of destination addresses, each corresponding to a different class of multicast communication, referred to as the multicast group id. A flag elsewhere in the header of a packet indicates that the message is a multicast rather than a unicast packet. When a multicast packet is received at a ring station, the multicast group id and the source node address, which is also in the header, are examined and based on those values and on the address of the receiving node, the packet may be replicated and sent on one or more output ports of the ring station according to the ring stations routing tables. Therefore, a different pattern of multicast can be configured for each different multicast group id. Note that acknowledgements are not required. The configuration of the multicast pattern is distributed among the ring stations, each of which has a limited view of the overall routing pattern.

Inside each client module (e.g., a computational subsystem), interface hardware monitors "writes" from the ring station and based on pre-specified writes generates signals (e.g., flags, event codes) that may initiate appropriate computations within the client, for example, as a result of polling the generated signals.

In addition to the toroidal-mesh interconnect network, the system can optionally have additional other interconnect topologies for different patterns of communication. For instance, certain global quantities such as the temperature or pressure of the system may need to be computed. Global communication patterns may also be required to synchronize the nodes. Options for the additional topologies include a low-latency but not necessarily high-bandwidth tree structure. In the present implementation, routing protocols are layered on top of the mesh interconnect to provide a virtual topology of the required type for the different communication patterns.

Each subsystem at a node in general includes its own internal memories, which are separate from the memory 3954. The memory controller 3950 includes a cache (e.g., that uses fast static RAM), which for many simulation scenarios is sufficiently large to hold the data for all the particles being handled by the node. The memory 3954 includes a larger storage space, which may be DRAM, which can be used for larger problems where each node is responsible for more particles. The interchange of data between the SRAM cache and the DRAM is coordinated in a buffering arrangement in which data in one buffer is being transferred while data in another buffer in the SRAM is being used for a computation.

For many of simulated systems (e.g., those of dimensions roughly 128Å cubed, or smaller), it is expected that the memory 3954 managed by the memory controller 3950 will not necessarily be used. In these cases, the primary purpose of the memory controller is the accumulation of force data. The cache of the memory controller is designed for high bandwidth accumulation of force data produced by the midrange or distant interactions in the midrange subsystems and the bond terms and corrections produced by the flexible subsystems. The memory subsystem of any specific node is responsible for accumulating force data for the particles owned by that node.

For large scale systems, the memory 3954 includes 1 to 2GB of DRAM at each node. In modes that use this DRAM, the system may be partitioned system is sub-partitioned-each box partitioned into sub-boxes—and the algorithms described above are implemented on a sub-box by sub-box basis, with the required sub-boxes being pulled out of DRAM to the memory controller cache as necessary. Note also that such a sub-box mode can in fact be used on smaller systems, before data exceeds the capacity of the memory controller cache, and there may be cases where this yields a performance advantage.

The subsystems described above and shown in FIG. 7 cooperate in the evaluation of a force field, the parameters of which are provisioned at the beginning of any simulation run. At the beginning of a simulation, the parameters of the force field, together with the description of the system-the particles (e.g., atoms) involved, their connectivity (e.g., covalent bonds), their positions in space and their current velocities-are all known and partitioned appropriately among nodes of the system. Specifically, the particle data (positions and velocities) reside in the flexible subsystem of an particle's homebox. In addition, each bonded term has an "owner" node, responsible for its computation.

The flexible subsystem 3912 is the "choreographer" of each simulation timestep. While most of the remaining subsystems are responsible for computation of the force field, the flexible subsystem implements the integration algorithm, which computes accelerations from forces, and increments velocities and positions incrementally from timestep to timestep. The integrator that is implemented for any particular type of simulation may require more than just simple integration: it may, for example, implement a constant temperature simulation-which generally requires the periodic calculation of temperature throughout the simulation. This in turn requires calculation of kinetic energy from velocity data, and the synthesis of a global scalar, the temperature, from this data. Such a particular computation, and others like it (pressure control, for example), are implemented by programming the flexible subsystem for these specific tasks; in this particular example, the flexible subsystems of all nodes must cooperate in computing a global reduction to compute this global scalar.

Before continuing with a detailed discussion of the overall computation timestep, or the architecture of the flexible subsystem, it is useful to consider the function and implementation of the midrange subsystem 3914.

The midrange subsystem 3914 can be thought of as a generic implementation of the NT Method that supports a much broader range of algorithms and decompositions. The subsystem is designed to efficiently calculate all pairwise interactions among point objects, whether particles or grid points, locally within a specified cutoff radius. One of its chief tasks is the computation of midrange non-bonded interactions through full pairwise summation. It is also used in the real-space components of the distant force calculation, for charge spreading and force interpolation.

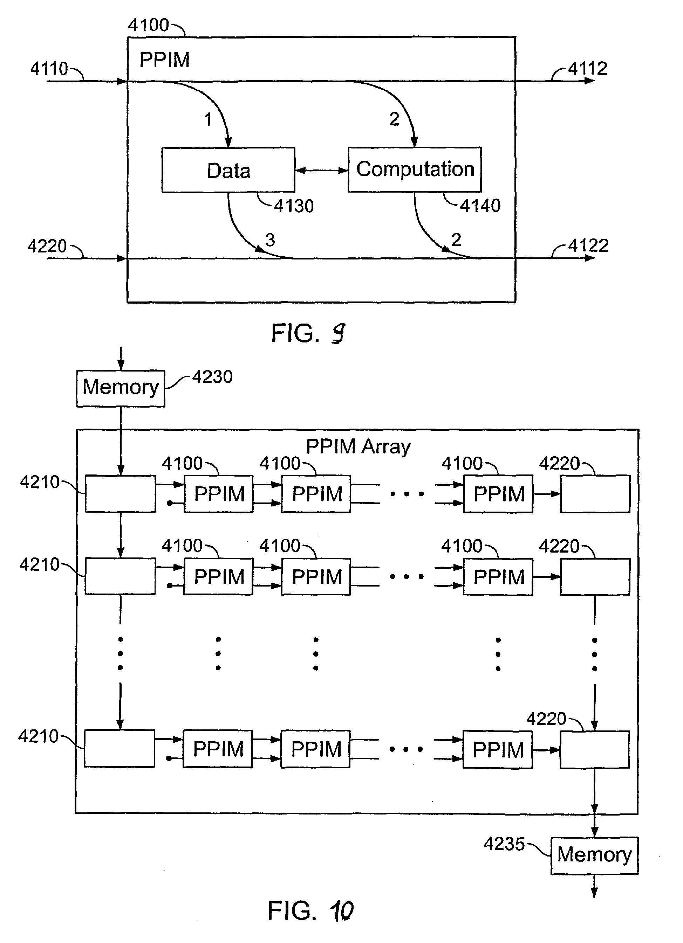

Referring to FIG. 9, operation of the midrange subsystem can be understood by considering a single particle-particle interaction module (PPIM) 4100. Generally, for computation of interactions between particles, during each iteration, memory in PPIM is first loaded with particle data for one set of particles at the beginning of the iteration. Then, particles in another set are streamed past the PPIM producing force contributions on the particles being streamed past. At the end of the iteration, force contributions for the particles that were resident in the PPIM is streamed out.

In some examples of computations performed by a PPIM, the PPIM operates in three phases. In the first phase (indicated by the circled numeral 1 in the figure), data for a first set of points, such as particles in the "tower" region for the NT method, are received over an input 4110 and stored in a memory 4130 of the PPIM. In a second phase, data for a second set of points, such as for particles in the "plate" region for the NT method, are received over input 4110 and passed to a computation element 4140. For each received particle, the computation element computes the total force (or other accumulation of pairwise computed quantities) on the particle from all stored particles, which were loaded in the first phase. This accumulated force is passed on an output 4122. In this second phase, there is one force send on output 4122 for each particle received on input 4110, with the ordering preserved. During the second phase, forces on the particles stored in memory 4130 are also accumulated. In the third phase, these forces are sent on output 4122, with one force sent for each particle receive on input 4110 in the first phase.

The PPIM includes storage for a number, for example three, different computations. In this way, different computations can be performed by the PPIM without reloading data, for example, allowing the first phase (data loading) phase of a second computation to be performed during the third phase (result unloading) phase of a first computation.

Referring to FIG. 10, the midrange subsystem implements a degree of parallelism by replicating the PPIM 4100 illustrated in FIG. 9 so that each PPIM performs only a subset of the required computations. In general, during the first phase, each PPIM receives only a portion of the first set of particle data, and in the second phase receives all the data in the second set. The outputs of all the PPIMs are then merged to yield the same result as would have been obtained by a single PPIM acting serially.

The midrange subsystem includes an array PPIMs 4100, as well as memories on the input 4230 and output 4235 of the array. The PPIMs are arranged into multiple rows, each with multiple PPIMs. In the present implementation, each row has eight PPIMs and there are four rows, but other arrangements can also be used.

Considering first one row of PPIMs, each PPIM passes data on its input 4110 (see FIG. 9) to its output 4112 without modification so that in the second phase of computation, each PPIM in a row receives data for the same set of particles. During the first phase, the data for the particles in the first set are also passed from PPIM to PPIM, but each PPIM stores only a separate portion of the data. During the second phase, the output 4122 of each PPIM includes one accumulated force for each particle input on its input 4110. At each PPIM (after the first, left-most) the PPIM receives a partially accumulated force for each particle on its input 4220, and internally sums the input contribution for that particle with the contribution computed by its computation element 4140, so that the output 4122 again has one force for each particle on its input 4110. Therefore, each row of PPIMs functions as a single PPIM, but performs more computations in parallel and therefore achieves higher throughput.

During the third phase, each PPIM streams out the accumulated forces for each point in the first set that was loaded to that PPIM, with "downstream" PPIMs in the row passing that force data without modification, with the overall order produced by the row matching the order the particles were loaded into the PPIM row during the first phase.

Considering the multiple rows of PPIMs, the data for the first set of particles is partitioned as it is read in so that each row has a separate portion of the first set. The data is passed through a set of distribution units 4210 to achieve this partitioning. The result is that each PPIM holds in its memory 4130 a different portion of the first set of points, with the PPIMs together holding all the points.

During the second phase, the distribution units 4210 replicate the particle data such that the same sequence of particles are sent to each row of PPIMs, and therefore also to each PPIM in that row. This results in each row of PPIMs producing a force for each particle in the second set, and these forces must be accumulated across the rows. This accumulation is performed by the accumulation units 4220.

During the third phase, the accumulation units 4220 concatenate the force data for the particles in the first set, so that at the output of the last accumulation unit 4220, the forces for the particles in the first set exit in the same order as the particle data was passed to the array in the first phase.

The midrange subsystem includes a front-end memory 4320, which received the particle data multicast from the flexible subsystems. A controller (not shown) monitors the writes to this memory and coordinates the passing of data to the array when the required data is available. The subsystem includes a back-end memory 4235, which is used to stage the computed forces (or other data). Note that this data is sent to the memory subsystem without waiting for completion of the processing so that some forces are being sent to the memory while other forces have not yet been computed.

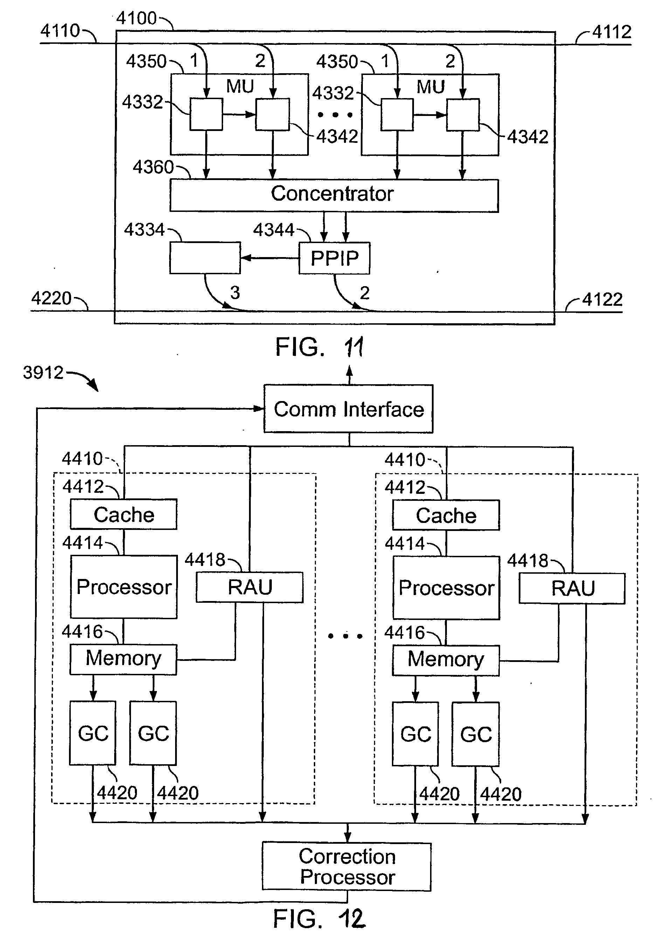

Referring to FIG. 11, each PPIM implements a further level of parallelism. Each PPIM contains several matchmaking units (MUs) 4350. In the present implementation, each PPIM includes eight MUs. The particle data distributed to the PPIM during the first phase, described above as being stored in memory 4130 (see FIG. 9), is actually distributed to memories 4332 in the MUs. That is, the particles in the first set are partitioned among the MUs. During the second phase, all the particles received by the PPIM are steamed past each matchmaking unit. For each received particle in the second set, a computation element 4342 of an MU determines which points stored in that MU's memory 4332 should interactions be computed, based primarily (but not exclusively) on the distance between the particles. The computation element 4342 passes the selected pairs to a concentrator 4360, which receives such selected pairs from each of the MUs, and acts essentially as an arbitrator for passing the selected pairs in a serial manner to a particle-particle interaction pipeline (PPIP) 4344. The PPIP then computes either the force on each particle due to the other or the potential energy contribution from the pair. The PPIP can be configured to compute a rather general function of the particles, based on their identities and inter-particle distance, and calculated by table-lookup and interpolation. For example, the PPIP is implemented or preconfigured to support a number of different functional forms, with data loaded into the PPIP determining the specific function within that functional form. The PPIP accumulates the forces for each particle in the second set for different of the stored particles, and passes the accumulated forces on output 4122 while storing the forces on the stored particles in a memory 4334. In the third phase, these stored forces in memory 4334 are streamed out on output 4122.

In some implementations of the PPIP, the functions that can be computed by the PPIP are represented as piecewise analytic functions. That is, within each of a set of ranges of argument value, a parametric analytic function is used to determine the function of the argument. A data structure stores the parameters of the analytic functions for the corresponding different ranges of argument values. A variable precision approach is used such that in different sizes of ranges of argument value can correspond to different parameters of the analytic function, for example, giving higher precision or greater accuracy in some ranges of argument value than in others. The selection of coefficients is performed in two stages. First, the argument range is divided into $N$ coarse ranges, each of which can have a different size. Each of the coarse ranges is divided into the same number $n$ of subranges, such that within each coarse range the subranges have the same size. For a particular input argument, first the coarse range is determined, and then the subrange within that coarse range is determined in order to look up the appropriate parameters of the analytic function. Therefore, there are $N$ times $n$ sets of coefficients. Given a limited number of sets of coefficients that can be used, the accuracy of the function to an ideal function can be chosen according to the argument range. An example of such a ideal function is erfc(1/R) and an example of the analytic functions are quadratic functions.

The PPIM array of the midrange subsystem in also used for charge spreading and force interpolation computations of the GSE methods described earlier in this document. For both charge spreading and force interpolation (the first and last thirds of the distant computation) particles are distributed to the PPIMs in a first phase, and the grid locations are streamed past the PPIMs in a second phase. Note that grid locations for the homeboxes of neighboring nodes streamed, as well as grid locations of the current node's homebox. Note that for this computation using an NT method, a "full plate" is needed because there is an ordering for the particle interaction so two equal and opposite forces are not computed at one time. During the charge spreading, no data needs to be accumulated by the PPIMs and streamed out in the third phase, while in force interpolation, the PPIMs do not need to provide outputs during the second phase, with the interpolated forces being accumulated during the second phase and streamed out of the PPIMs in the third phase.

The flexible subsystem 3912 also implements parallelism within the subsystem. Referring to FIG. 12, a groups of computational elements 4410 function relatively independently in parallel. Not only is each particle assigned to a particular node within the system (i.e., the node associated with the homebox holding the particle) each particle is assigned to a particular group 4410 within the flexible subsystem of that node. In the present implementation, there are four groups of computation elements 4410.

Each group 4410 includes a processor 4414. Each processor has data and instruction caches (32KB) 4412 that are backed by the memory system accessed over the communication ring. In many applications the caches 4412 are sufficiently large to be able to hold all the data needed for an iteration. Each processor has a remote access unit (RAU) 4418, which implements functions including direct memory access (DMA) functions for transferring data from the memory over the communication ring to a memory 4416, which is accessible to the processor 4414. The particle data that is the responsibility of processor is held in memory 4416, and the RAU 4418 retrieves force data for those particles from the memory subsystem using a DMA approach without loading the processor 4414. The RAU is coupled to a ring station through a communication interface, which aggregates outputs and distributes inputs to the flexible subsystem to the appropriate elements (e.g., based on the addresses of received packets).

Each processor has a number of slave special-purpose co-processors 4420, referred to below as "geometry cores" (GCs). In the present implementation, there are two GCs per processor. Each GC has a four-way SIMD architecture, dual issue VLIW pipelined ALU, and includes "geometric" primitives including computation of inner ("dot") products. Each GC uses a vector/scalar register architecture in which registers can be addressed in an interleaved manner as either n vector registers or as 4n scalar registers, and where scalar results can be placed directly in a portion of a register where needed for subsequent computations. There are inbound queues for the GCs that are fed by its processor, and outputs from the GC can be fed back to the memory system without requiring handling by the processor..

The GCs are used to compute bonded force terms. Each bond is assigned to a particular GC, and the processors exchange particle position data so that each processor has the data it needed to have its GC compute bonded force contributions. Note that a processor that computes a bonded force contribution may not be responsible for any of the particles in that bonded pair. Periodically (e.g., every 2000 time steps) a reassignment of bonds to processors and GCs may be performed as an optimization that may reduce communication requirements.

In a computation iteration, particle position data is distributed among the memories 4416 of the processors of the flexible subsystems of the nodes of the overall system. From there, the particle locations are sent to the midrange subsystems for computation of the pairwise interactions, and to other processors for computation of bonded terms..

The flexible subsystem 3912 also includes a correction processor 4430 which is used to add correction terms to values computed by the GCs. The correction processor implements an example of a general computational approach. In its general form, if one computation is to be performed on a large part A of a data set and a second computation is to be performed on a small part b of the data set, where A and b form a partition of the complete data set, and the results of the computations are to be accumulated (or more generally reduced) over the data set, then it may be efficient to perform and accumulate the first computation over the entire data set, and then for the small part b recompute and subtract the result of the first computation and compute and add the result of the first computation. For example, by not having to make exceptions for elements of the data set in part b, the first computation may be made efficient by regular (i.e., condition or exception free) iteration. In the context of particle interactions the entire data set (i.e. A plus b) can correspond to a set of particle pairs that are within an interaction radius, the first computation can be a unbonded interaction, for example, as computed in the PPIM array, and the second computation can be a bonded interaction as computed in the GCs. So rather than having to exclude bonded pairs of particles that are within the interaction radius from force computations in the PPIM array, when a bonded interaction between particles is computed a GC, the correction processor 4430 computes the negative to the unbonded term that is computed in the PPIM for that pair of particles, and adds it to the bonded term before it is sent to the memory where it is accumulated. In some implementations, the correction processor includes the same computational structure as a PPIP 4344, further ensuring that exactly the same unbonded term is computed for subtraction from the total.

There are generally three major computations that are hosted in the flexible subsystem: bonded force terms, constrain terms and integration (performed without the constraints), and finally updated positions accounting for the constraints. During the computation of distant forces, the processors and the GCs also perform performing the FFT calculations in versions of the system that do not include a dedicated FFT subsystem.

In versions of the system that use the flexible subsystem to perform the FFT computations, the distant forces are computed by first distributing charges to the grid locations using the midrange subsystem. These distributed charges are returned to the flexible subsystem, which implements a distributed FFT computation for the grid spanning the entire simulation volume. The FFT is distributed among the processors (and their associated GCs) and requires multiple phases of exchange of intermediate results between the nodes. At the end of the FFT iteration, the transformed data, which represents potentials at grid locations, is passed back to the midrange subsystem, where it is used to compute interpolated forces on the particles. These interpolated forces are then sent to the memory subsystem whether the distant force terms are combined with the other force terms computed for each particle.

In the present system, each node is implemented using an application specific integrated circuit (ASIC) that includes all the computation and communication subsystems shown in FIG. 7, with the exception of the memory 3954 and remote ring stations 3930. Multiple ASICs are arranged one each of multiple circuit boards that are interconnected through a backplane. The internode communication links 3730 are therefore either local to a circuit board or are routed through the backplane.

## 4 Applications

The algorithmic techniques, or the hardware or software techniques, described above may be applicable to a variety of applications, some of which are related to biomolecular simulation while others are not. The applications may include, for example, the following:

Biomolecular simulation applications related to use of molecular dynamics, Monte Carlo simulation, or other sampling technique, can include without limitation: ab initio protein structure prediction, refinement of homology models of protein structure, modeling of protein folding kinetics, computation of protein-ligand binding free energies, determining whether a pair of proteins will interact; determining their binding site and their binding free energy; toxicology studies; computation of hydration free energies; protein design; modeling allosteric changes and protein function; simulation of membrane proteins; simulation of nucleic acids; simulation of carbohydrates; simulation of combinations of the above and interactions between them; predicting membrane penetration, including blood-brain barrier penetration; molecular force field development; and simulations of crystal structures

Applications may also involve other fields, such as material science applications, weather simulations, hydrodynamic simulations, equation of state simulations, astrophysics simulations, etc.

## 5 Alternatives

A variety of arrangements of distributed processing nodes can alternatively be used with the computation decompositions described above.

It is also not necessary that there be a one-to-one correspondence between processing nodes and homeboxes. For example, each node may be responsible for a set of homeboxes, such as a $n \times n \times n$ cube of homeboxes. One use of such a finer-grain definition of homeboxes is in multiple-zone scheduling approaches in which the schedule of zone-to-zone interactions can be used to avoid a need to test for duplicate computations or can be used to reduce the amount of communication through improved rounding. Another use of such finer-grain homeboxes is in serializing the computation involved with each pair of interacting zones to make better use of local memory, such as local cache memory, at each of the processing nodes. Aspects of memory utilization are discussed further below in the context of serial implementations.

In one alternative version of a parallel implementation, each node maintains data for more particles than those that are located within its homebox. A "clone buffer" approach is used in conjunction with a midpoint assignment rule approach. In particular, each node stores particle data from a region that extends a distance $R/2$ beyond the boundaries of its homebox. After each computation phase, the forces for all the particles in the extended region are sent to that node allowing it to redundantly compute the new locations of the particles. During the computation phase, further importing of particle data is not necessary, for example, using the midpoint assignment rule or the pseudo-random assignment rule, because the extended region includes all the particles needed.

The approaches described above can be implemented in software, in hardware, or using a combination of hardware and software. For example, alternative versions of the system can be implemented in software, in firmware, in digital electronic circuitry, in computer hardware, in other modes of implementation, or in combinations of such modes. The system can include a computer program product tangibly embodied in a machine-readable storage device, such as on a magnetic or an optical disc or in a semiconductor memory, for execution by a programmable processor or by a system that includes multiple programmable processors. The programmable processors can be general-purpose processors, or can be special-purpose processors, for example, designed for computations of the types described above. Parallel implementation can, for example, use special-purpose interconnection networks, or can use more general purpose data networking approaches. Method steps can be performed by a programmable processor executing a program of instructions to perform functions by operating on input data and generating output. The system can be implemented in one or more computer programs that are executable on a programmable system including at least one programmable processor coupled to receive data and instructions from, and to transmit data and instructions to, a data storage system, at least one input device, and at least one output device. Each computer program can be implemented in a high-level procedural or object-oriented programming language, or in assembly or machine language if desired. Such language can be either a compiled or interpreted language. Suitable processors include, by way of example, either (or both) general and special purpose microprocessors. Generally, a processor will receive instructions and data from read-only memory and/or random access memory. Generally, a computer will include one or more mass storage devices for storing data files. Such devices include magnetic disks such as internal hard disks and removable disks, magneto-optical disks, or optical disks. Storage devices suitable for tangibly embodying computer program instructions and data include all forms of non-volatile memory, including by way of example semiconductor memory devices such as EPROM, EEPROM, and flash memory devices; magnetic disks such as internal hard disks and removable disks; magneto-optical disks; and CD-ROM disks. Any of the foregoing can be incorporated in or supplemented application-specific hardware, such as application-specific integrated circuits (ASICs).

Other embodiments are also within the scope of the appended claims. Reference numerals in the claims, which are provided in parentheses to increase intelligibility, should not be construed as limiting the claim.

**Claims**

1. A computation system for computing interactions in a multiple-body simulation comprising an array of a plurality of processing modules arranged into one or more processing groups, each including a plurality of serially interconnected processing modules, **characterized by**:

    each of the processing modules (4100) including a storage (4130) for data elements and including circuitry (4140) for performing pairwise computations between data elements, each associated with a spatial location, each of the pairwise computations making use of a data element from the storage of the processing module and a data element passing through the serially interconnected processing modules; and
    each of the processing modules including circuitry (4350) for selecting pairs of data elements according to separations between spatial locations associated with the data elements.

2. The system of claim 1 wherein the array of processing modules includes two or more processing groups.

3. The system of claim 1 further comprising distribution modules (4210) for distributing data elements among the processing groups.

4. The system of claim 1 further comprising combination modules (4220) for combining results of the pairwise computations produced by each of the processing groups.

5. A processing module (4100) for an array of a plurality of serially interconnected processing modules for computing particle interactions in a multiple-body simulation, the module being configured to perform pairwise computations between data elements each associated with a corresponding spatial location, each of the pairwise computations using one data element from a first set of data elements and one data element from a second set of data elements, the module comprising:

    a plurality of matching units (4350), each unit being associated with a storage for a different part of a first set of data elements, each unit being configured to select pairs of data elements according to separations between the locations associated with the data elements, wherein for each pair, one data element is from the first set and one data element is from a second set of data elements;
    an input (4110) for receiving from another processing module of said plurality of serially interconnected processing modules a series of data elements of the second set of data elements, the input being coupled to each of the matching units for passing the received data elements to the matching units;
    a computation unit (4344) coupled to the matching units for receiving pairs of data elements selected by the matching units according to the separations; and
    an output (4122) for transmitting results produced by the computation unit.

6. The module of claim 5 further comprising:

    an output (4112) for transmitting the received series of data elements to another module.

7. The module of claim 5 further comprising:

    an input (4220) for receiving results produced by other modules; and
    a merging unit for combining the results produced by the computation unit and the results received on the input for receiving results.

8. The module of claim 7 wherein the merging unit includes circuitry for merging results according to a data element of the first set that is associated with each of the results.

9. A method for computing interactions in a multiple-body simulation, the method comprising:

receiving a first set of data elements, each of said data elements being associated with a corresponding spatial location;

distributing the first set of data elements to storages of an array of a plurality of processing modules (4100) that are arranged into one or more processing groups, each including a plurality of serially interconnected processing modules, including storing a different part of the first set of data elements at each of the processing modules;

receiving a second set of data elements, each of said data elements being associated with a corresponding spatial location;

passing the received data elements through the serially interconnected processing groups; at each of at least some of the processing modules

selecting some but not all of the received data elements for the performing of the pairwise computations according to separations between spatial locations associated with the received data elements and spatial locations associated with the data elements distributed to the processing module,

performing pairwise computations each involving a data element from the first set that is stored in the storage of the processing module and a data element from the second set passing through the processing group, and passing a result of the pairwise computations through the serially interconnected processing group;

combining the results of the pairwise computations passed from each of the processing groups; and transmitting the combined results.

10. The method of claim 9 wherein performing each pairwise computation includes performing a computation for data elements that are associated with simulated bodies in the multiple-body simulation.

11. The method of claim 10 wherein the pairwise computations performed at the processing modules are associated with forces between pairs of the bodies.

12. The method of claim 9 further comprising, at each of the processing nodes, accumulating results of the pairwise computations in association with data elements of the first set stored at said processing node.

13. The method of claim 12 wherein passing the result of the pairwise computations includes passing the accumulated results of said computations.

14. The method of claim 9 wherein distributing the first set of data elements to the storages of the array includes, at each of the modules, distributing data elements to a plurality of matching units, each matching unit receiving a different part of the first set of data elements.

15. The method of claim 14 wherein the method further comprises at each of the at least some of the processing modules at each of the matching units selecting some but not all of the data elements data elements passing through the processing group for the performing of the pairwise computations.

16. The method of claim 15 wherein the method further comprises at each of the at least some of the processing modules combining the outputs of the matching units, and passing the combined outputs to a computation unit for executing the pairwise computation.

17. A software product adapted to perform all the steps of any one of claims 9-16 when the software is run on a computer.

18. The software product of claim 17 including instructions for performing the steps embodied on a computer-readable medium.

**Patentansprüche**

1. Berechnungssystem zur Berechnung von Wechselwirkungen in einer Mehr-Körper-Simulation mit einem Feld aus einer Vielzahl von Verarbeitungsmodulen, die in einer oder mehreren Verarbeitungsgruppen angeordnet sind, die jeweils eine Vielzahl von seriell verbundenen Verarbeitungsmodulen umfassen, **dadurch gekennzeichnet, dass**:

jedes der Verarbeitungsmodule (4100) einen Speicher (4130) für Datenelemente umfasst und Schalttechnik (4140) zur Durchführung paarweiser Berechnungen zwischen Datenelementen, denen jeweils eine räumliche Position zugeordnet ist, umfasst, wobei jede der paarweisen Berechnungen von einem Datenelement aus dem Speicher des Verarbeitungsmoduls und von einem durch die seriell verbundenen Verarbeitungsmodule übergebenen Datenelement Gebrauch macht; und

jedes der Verarbeitungsmodule Schalttechnik (4350) zur Auswahl von Paaren von Datenelementen gemäß den Trennungen zwischen den räumlichen Position, die den Datenelementen zugeordnet sind, umfasst.

2. System gemäß Anspruch 1, wobei das Feld aus Verarbeitungsmodulen zwei oder mehr Verarbeitungsgruppen umfasst.

3. System gemäß Anspruch 1, ferner mit Verteilungsmodulen (4210) zur Verteilung von Datenelementen unter den Verarbeitungsgruppen.

4. System gemäß Anspruch 1, ferner mit Kombinationsmodulen (4220) zur Kombination von Ergebnissen der paarweisen Berechnungen, die von jeder der Verarbeitungsgruppen erzeugt sind.

5. Verarbeitungsmodul (4100) für ein Feld aus einer Vielzahl von seriell verbundenen Verarbeitungsmodulen zur Berechnung von Wechselwirkungen von Teilchen in einer Mehr-Körper-Simulation, wobei das Modul eingerichtet ist, um paarweise Berechnungen zwischen Datenelementen, denen jeweils eine entsprechende räumliche Position zugeordnet ist, durchzuführen, wobei jede der paarweisen Berechnungen ein Datenelement aus einem ersten Satz von Datenelementen und ein Datenelement aus einem zweiten Satz von Datenelementen verwendet, wobei das Modul aufweist:

eine Vielzahl von Abgleicheinrichtungen (4350), wobei jeder Einrichtung ein Speicher für einen verschiedenen Teil eines ersten Satzes von Datenelementen zugeordnet ist, und wobei jede Einrichtung eingerichtet ist, um Paare von Datenelementen gemäß den Trennungen zwischen den Positionen, die den Datenelementen zugeordnet sind, auszuwählen, wobei für jedes Paar ein Datenelement aus dem ersten Satz stammt und ein Datenelement aus einem zweiten Satz von Datenelementen stammt;
einen Eingang (4110) zum Empfang einer Reihe von Datenelementen des zweiten Satzes von Datenelementen von einem weiteren Verarbeitungsmodul aus der Vielzahl von seriell verbundenen Verarbeitungsmodulen, wobei der Eingang zur Übergabe der empfangenen Datenelemente an die Abgleicheinrichtungen an jede der Abgleicheinrichtungen gekoppelt ist;
eine Berechnungseinrichtung (4344), die zum Empfang von Paaren von Datenelemente, die durch die Abgleicheinrichtungen gemäß den Trennungen ausgewählt sind, an die Abgleicheinrichtungen gekoppelt ist; und
einen Ausgang (4122) zur Übertragung von Ergebnissen, die von der Berechnungseinrichtung erzeugt sind.

6. Modul gemäß Anspruch 5, ferner mit:

einem Ausgang (4112) zur Übertragung der empfangenen Reihe von Datenelemente an ein weiteres Modul.

7. Modul gemäß Anspruch 5, ferner mit:

einem Eingang (4220) zum Empfang von Ergebnissen, die von anderen Modulen erzeugt sind; und
einer Mischeinrichtung zur Kombination der Ergebnisse, die von der Berechnungseinrichtung erzeugt sind, und der Ergebnisse, die an dem Eingang zum Empfang von Ergebnissen empfangen sind.

8. Modul gemäß Anspruch 7, wobei die Mischeinrichtung Schalttechnik umfasst zur Mischung von Ergebnissen gemäß einem Datenelement des ersten Satzes, dem das jeweilige der Ergebnisse zugeordnet ist.

9. Verfahren zur Berechnung von Wechselwirkungen in einer Mehr-Körper-Simulation, wobei das Verfahren aufweist:

Empfangen eines ersten Satzes von Datenelementen, wobei jedem der Datenelemente eine entsprechende räumliche Position zugeordnet ist;
Verteilen des ersten Satzes von Datenelementen an Speicher aus einem Feld aus einer Vielzahl von Verarbeitungsmodulen (4100), die in eine oder mehrere Verarbeitungsgruppen angeordnet sind, wobei jede eine Vielzahl von seriell verbundenen Verarbeitungsmodulen umfasst, wobei das Verteilen ein Speichern eines verschiedenen Teils des ersten Satzes von Datenelementen in jedem der Verarbeitungsmodule umfasst;
Empfangen eines zweiten Satzes von Datenelementen, wobei jedem der Datenelemente eine entsprechende räumliche Position zugeordnet ist;

Übergeben der empfangenen Datenelemente durch die seriell verbundenen Verarbeitungsgruppen;
an jedem von zumindest einigen der Verarbeitungsmodulen

Auswählen einiger aber nicht aller der empfangenen Datenelemente zum Durchführen der paarweisen Berechnungen gemäß den Trennungen zwischen räumlichen Positionen, die den empfangenen Datenelementen zugeordnet sind, und räumlichen Positionen, die den Datenelementen zugeordnet sind, die an das Verarbeitungsmodul verteilt sind,

Durchführen von paarweisen Berechnungen von denen jede ein Datenelement aus dem ersten Satz, der in dem Speicher des Verarbeitungsmoduls gespeichert ist, und

ein Datenelement aus dem zweiten Satz, der durch die Verarbeitungsgruppe übergeben ist, einbezieht, und
Übergeben eines Ergebnisses der paarweisen Berechnungen durch die seriell verbundene Verarbeitungsgruppe;

Kombinieren der Ergebnisse der paarweisen Berechnungen, die von der jeweiligen der Verarbeitungsgruppen übergeben sind; und
Übertragen der kombinierten Ergebnisse.

10. Verfahren gemäß Anspruch 9, wobei ein Durchführen jeder paarweisen Berechnung ein Durchführen einer Berechnung für Datenelemente umfasst, denen simulierten Körper in der Mehr-Körper-Simulation zugeordnet sind.

11. Verfahren gemäß Anspruch 10, wobei den an den Verarbeitungsmodulen durchgeführten paarweisen Berechnungen Kräften zwischen Paaren der Körper zugeordnet sind.

12. Verfahren gemäß Anspruch 9, ferner mit einem Anhäufen von Ergebnissen der paarweisen Berechnungen in Verbindung mit Datenelementen des ersten Satzes, der an dem Verarbeitungsknoten gespeichert ist, an jedem der Verarbeitungsknoten.

13. Verfahren gemäß Anspruch 12, wobei das Übergeben des Ergebnisses der paarweisen Berechnungen ein Übergeben der angehäuften Ergebnisse der Berechnungen umfasst.

14. Verfahren gemäß Anspruch 9, wobei ein Verteilen des ersten Satzes von Datenelementen an die Speicher aus dem Feld ein Verteilen von Datenelementen an eine Vielzahl von Abgleicheinrichtungen an jedem der Module umfasst, wobei jede Abgleicheinrichtung einen verschiedenen Teil des ersten Satzes von Datenelementen empfängt.

15. Verfahren gemäß Anspruch 14, wobei das Verfahren ferner an jedem der zumindest einigen der Verarbeitungsmodule an jeder der Abgleicheinrichtungen, die einige aber nicht alle der Datenelemente auswählen, ein Übergeben von Datenelementen durch die Verarbeitungsgruppe zum Durchführen der paarweisen Berechnungen aufweist.

16. Verfahren gemäß Anspruch 15, wobei das Verfahren ferner an jedem der zumindest einigen der Verarbeitungsmodule ein Kombinieren der Ausgänge der Abgleicheinrichtungen und ein Übergeben der kombinierten Ausgänge an eine Berechnungseinrichtung zum Ausführen der paarweisen Berechnung umfasst.

17. Softwareprodukt, das angepasst ist, um alle Schritte gemäß einem der Ansprüche 9 bis 16 durchzuführen, wenn die Software auf einem Computer ausgeführt wird.

18. Softwareprodukt gemäß Anspruch 17, das Anweisungen zum Durchführen der Schritte umfasst, das auf einem computerlesbaren Datenträger ausgeformt ist.

**Revendications**

1. Système de calcul pour calculer des interactions dans une simulation à corps multiples comprenant une matrice d'une pluralité de modules de traitement agencés dans un ou plusieurs groupes de traitement, chacun comprenant une pluralité de modules de traitement interconnectés en série, **caractérisé par** :

chacun des modules de traitement (4100) comprenant un stockage (4130) pour des éléments de données et comprenant un ensemble de circuits (4140) pour effectuer des calculs par paires entre des éléments de données, chacun associé à une localisation spatiale, chacun des calculs par paires utilisant un élément de données du stockage du module de traitement et un élément de données passant à travers les modules de traitement interconnectés en série ; et

chacun des modules de traitement comprenant de la circuiterie (4350) pour sélectionner des paires d'éléments de données selon des séparations entre des localisations spatiales associées aux éléments de données.

2. Système selon la revendication 1, dans lequel la matrice de modules de traitement comprend deux groupes de traitement ou plus.

3. Système selon la revendication 1, comprenant en outre des modules de distribution (4210) pour distribuer des éléments de données entre les groupes de traitement.

4. Système selon la revendication 1, comprenant en outre des modules de combinaison (4220) pour combiner des résultats des calculs par paires produits par chacun des groupes de traitement.

5. Module de traitement (4100) pour une matrice d'une pluralité de modules de traitement interconnectés en série pour calculer des interactions de particules dans une simulation à corps multiples, le module étant configuré pour effectuer des calculs par paires entre des éléments de données chacun associé à une localisation spatiale correspondante, chacun des calculs par paires utilisant un élément de données d'un premier ensemble d'éléments de données et un élément de données d'un second ensemble d'éléments de données, le module comprenant :

une pluralité d'unités de mise en concordance (4350), chaque unité étant associée à un stockage pour une partie différente d'un premier ensemble d'éléments de données, chaque unité étant configurée pour sélectionner des paires d'éléments de données selon des séparations entre les localisations associées aux éléments de données, où pour chaque paire, un élément de données provient du premier ensemble et un élément de données provient d'un second ensemble d'éléments de données ;

une entrée (4110) pour recevoir en provenance d'un autre module de traitement de ladite pluralité de modules de traitement interconnectés en série une série d'éléments de données du second ensemble d'éléments de données, l'entrée étant couplée à chacune des unités de mise en concordance pour passer les éléments de données reçus aux unités de mise en concordance ;

une unité de calcul (4344) couplée aux unités de mise en concordance pour recevoir des paires d'éléments de données sélectionnées par les unités de mise en concordance selon les séparations ; et

une sortie (4122) pour transmettre des résultats produits par l'unité de calcul.

6. Module selon la revendication 5, comprenant en outre :

une sortie (4112) pour transmettre la série d'éléments de données reçue à un autre module.

7. Module selon la revendication 5, comprenant en outre :

une entrée (4220) pour recevoir des résultats produits par d'autres modules ; et

une unité de fusion pour combiner les résultats produits par l'unité de calcul et les résultats reçus sur l'entrée recevant les résultats.

8. Module selon la revendication 7, dans lequel l'unité de fusion comprend de la circuiterie pour fusionner les résultats selon un élément de données du premier ensemble qui est associé à chacun des résultats.

9. Procédé pour calculer des interactions dans une simulation à corps multiples, le procédé comprenant :

recevoir un premier ensemble d'éléments de données, chacun desdits éléments de données étant associé à une localisation spatiale correspondante ;

distribuer le premier ensemble d'éléments de données à des stockages d'une matrice d'une pluralité de modules de traitement (4100) qui sont agencés dans un ou plusieurs groupes de traitement, chacun comprenant une pluralité de modules de traitement interconnectés en série, la distribution comprenant le stockage d'une partie différente du premier ensemble d'éléments de données au niveau de chacun des modules de traitement ;

recevoir un second ensemble d'éléments de données, chacun desdits éléments de données étant associé à une localisation spatiale correspondante ;

passer les éléments de données reçus à travers les groupes de traitement interconnectés en série ;

au niveau de chacun d'au moins certains des modules de traitement

- sélectionner certains mais pas tous les éléments de données reçus pour l'exécution des calculs par paires selon des séparations entre des localisations spatiales associées aux éléments de données reçus et des localisations spatiales associées aux éléments de données distribués au module de traitement,
- exécuter des calculs par paires impliquant chacun un élément de données du premier ensemble qui est stocké dans le stockage du module de traitement et un élément de données du second ensemble passant à travers le groupe de traitement, et
- passer un résultat des calculs par paires à travers le groupe de traitement interconnecté en série ;

combiner les résultats des calculs par paires passés depuis chacun des groupes de traitement ; et
transmettre les résultats combinés.

**10.** Procédé selon la revendication 9, dans lequel l'exécution de chaque calcul par paires comprend l'exécution d'un calcul pour des éléments de données qui sont associés à des corps simulés dans la simulation à corps multiples.

**11.** Procédé selon la revendication 10, dans lequel les calculs par paires effectués au niveau des modules de traitement sont associés à des forces entre des paires des corps.

**12.** Procédé selon la revendication 9, comprenant en outre, au niveau de chacun des noeuds de traitement, l'accumulation de résultats des calculs par paires en association avec des éléments de données du premier ensemble stocké au niveau dudit noeud de traitement.

**13.** Procédé selon la revendication 12, dans lequel le passage du résultat des calculs par paires comprend le passage des résultats accumulés desdits calculs.

**14.** Procédé selon la revendication 9, dans lequel la distribution du premier ensemble d'éléments de données aux stockages de la matrice comprend, au niveau de chacun des modules, la distribution d'éléments de données à une pluralité d'unités de mise en concordance, chaque unité de mise en concordance recevant une partie différente du premier ensemble d'éléments de données.

**15.** Procédé selon la revendication 14, dans lequel le procédé comprend en outre au niveau de chacun des au moins certains des modules de traitement au niveau de chacune des unités de mise en concordance la sélection de certains mais pas de tous les éléments de données passant à travers le groupe de traitement pour l'exécution des calculs par paires.

**16.** Procédé selon la revendication 15, dans lequel le procédé comprend en outre au niveau de chacun des au moins certains des modules de traitement la combinaison des sorties des unités de mise en concordance, et le passage des sorties combinées à une unité de calcul pour l'exécution du calcul par paires.

**17.** Produit logiciel adapté pour effectuer toutes les étapes de l'une quelconque des revendications 9 à 16 lorsque le logiciel est exécuté sur un ordinateur.

**18.** Produit logiciel selon la revendication 17 comprenant des instructions pour effectuer les étapes, incorporé à un support lisible par ordinateur.

| Decomposition | Asymptotic Scaling |
|---|---|
| Atom | $O(1)$ |
| Force | $O(p^{-1/2})$ |
| Spatial | $O(R^3)$ |
| Shaw's Neutral Territory (NT) | $O(R^{3/2}p^{-1/2})$ |
| Snir's Hybrid (SH) | $O(R^{3/2}p^{-1/2})$ |

R is the Interaction Radius
p is the Number of Processors

FIG. 1

FIG. 2

FIG. 3

FIG. 4

3710

3710

3735    3720

3720

3730

3720

3740    Processor
3750    Local    Comm.
        MEM    Interface
                        3720

                        3760

# FIG. 5

3810

Velocity Integration

3820

Position Integration

3830

Force Calculation

3832

Bonded Force Calculations

3834

Midrange Force Calculations

3836

Distant Force Calculations

Force Accumulation

3838

FIG. 6

Node i

Node j

3912

Flexible Subsystem

3912

Flexible Subsystem

3914

Midrange Subsystem

3914

Midrange Subsystem

3954

Memory

3954

Memory

FIG. 8

FIG. 7

FIG. 9

FIG. 10

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006113825 A
- US 20060129363 A1
- US 20060142980 A1
- WO 2006004877 A2
- US 6600788 B

### Non-patent literature cited in the description

- **Heffelfinger.** Parallel atomistic simulations. *Computer Physics Communications,* 2000, vol. 128, 219-237
- **Plimpton.** Fast Parallel Algorithms for Short Range Molecular Dynamics. *Journal of Computational Physics,* March 1995, vol. 117, 1-19
- **Marc Snir.** A Note on N-Body Computations with Cutoffs. *Theory Comput. Systems,* 2004, vol. 37, 295-318